# EUROPÄISCHE PATENTSCHRIFT

(11) **EP 1 125 570 B1**
(45) Veröffentlichungstag und Bekanntmachung des Hinweises auf die Patenterteilung: **18.08.2004**
(21) Anmeldenummer: 00127510.6
(22) Anmeldetag: 15.12.2000
(51) Int. Cl.: A61F 13/20

(54) **Tampon**
Tampon
Tampon

(30) Priorität: 10.02.2000 DE 20002337 U
(43) Veröffentlichungstag der Anmeldung: 22.08.2001
(73) Patentinhaber: HYSALMA Hygiene Sales & Marketing GmbH, 45481 Mülheim an der Ruhr (DE)
(72) Erfinder:
(74) Vertreter: COHAUSZ DAWIDOWICZ HANNIG & PARTNER

(56) Entgegenhaltungen:
- EP-A- 0 611 562
- EP-A- 1 108 407
- US-A- 3 068 867
- US-A- 3 674 030
- US-A- 3 710 420
- US-A- 4 175 561

## Beschreibung

Die vorliegende Erfindung betrifft einen Tampon für die Frauenhygiene (Menstrualtampon) zum Einführen in die Scheide einer Frau mit einem aufsaugenden Material, vorzugsweise aus zellulosischen Fasern, und einer die Fasern umgebenden äußeren Hülle, wobei in der Außenseite Längsrillen angeordnet sind.

Bekannte Wickeltampons dehnen sich bei Flüssigkeitsaufnahme gleichmäßig aus und bilden eine runde Querschnittsform. Sie füllen daher nicht in allen Fällen die Vagina der Benutzerin aus. Bei stärkerem, insbesondere stoßweisem Flüssigkeitsaustritt kann es daher zu einer Leakage kommen, weil die Flüssigkeit in den nicht ausgefüllten Zwischenräumen am Tampon vorbeilaufen kann.

Aufgabe der Erfindung ist es, einen Tampon der eingangs genannten Art zu schaffen, der bei Verbesserung der Passform und der Produktsicherheit die Gefahr einer Leakage reduziert, ohne die Menge des Saugmaterials zu erhöhen.

Diese Aufgabe wird erfindungsgemäß dadurch gelöst, daß der Tampon sowohl vor dem Verpressen als auch im ausgedehnten vollgesogenen Zustand eine ovale Querschnittsform besitzt und daß der Tampon im nicht ausgedehnten zusammengepressten Zustand eine runde Querschnittsform besitzt.

Die ovale, insbesondere elliptische Ausdehnung des Tampons in der Körperhöhle führt dazu, daß der Tampon sich der Körperhöhle optimal anpasst und ein Vorbeffließen der Menstruationsflüssigkeit am Tampon sicher verhindert wird. Während des Einführens des Tampons ist dagegen dieser von runder Querschnittsform, so daß das Einführen nicht beeinträchtigt wird.

Vorzugsweise wird vorgeschlagen, daß der Tampon im nicht ausgedehnten zusammengepressten Zustand eine runde Querschnittsform besitzt. Dadurch ist er besonders leicht einführbar.

Durch eine ungerade Anzahl von Längsrillen wird die nicht runde Ausdehnung des Tampons noch unterstützt. Vorzugsweise sind sieben oder neun Rillen vorgesehen.

Ein Ausführungsbeispiel der Erfindung ist in der Zeichnung perspektivisch dargestellt und wird im folgenden näher beschrieben. Es zeigen:
- Figur 1: den Tamponrohling vor dem Verpressen
- Figur 2: den Tampon im gepressten Zustand und
- Figur 3: den Tampon im vollgesogenen Zustand.

Der Tampon 1 besteht vorzugsweise aus zellulosischen Fasern als aufsaugendes Material, das von einer äußeren glatten, Flüssigkeit durchlässigen Hülle 2 umgeben ist. Als Rohling weist der Tampon eine ovale, insbesondere elliptische Querschnittsform auf.

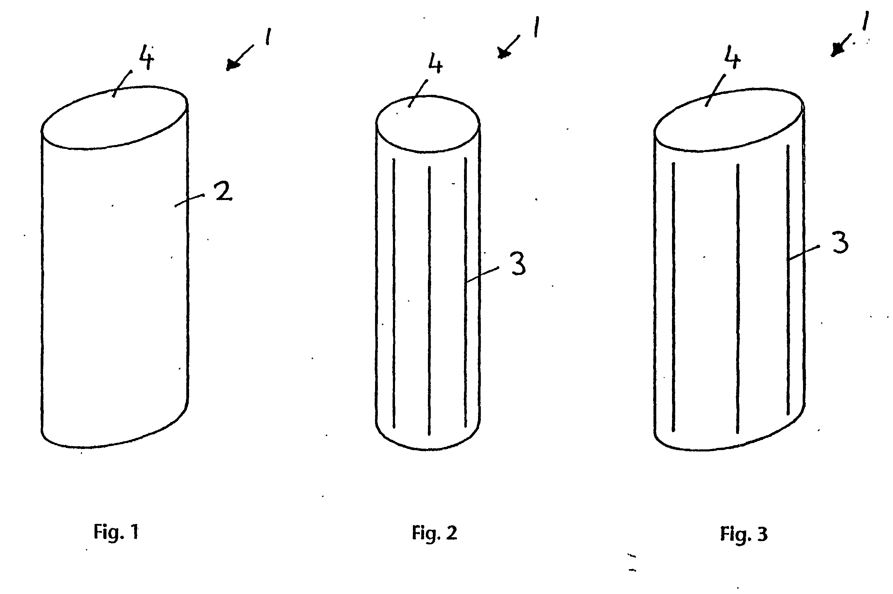

Der Rohling wird danach in eine zylindrische Form mit rundem Querschnitt gepresst (Figur 2), wobei in die Außenseite sieben oder neun Längsrillen 3 eingedrückt werden, die zueinander etwa gleich große Abstände haben.

Sobald der Tampon Flüssigkeit aufsaugt, nimmt er wieder in etwa den ausgedehnten Zustand an, wobei er dann wieder eine ovale Querschnittsform einnimmt.

Im gepressten Zustand (Figur 2) weist der Tampon an seinem vorderen Ende (Einführende) einen spitzen Tamponkopf auf.

## Patentansprüche

1. Tampon (1) für die Frauenhygiene (Menstrualtampon) zum Einführen in die Scheide einer Frau mit einem aufsaugenden Material aus zellulosischen Fasern und einer die Fasern umgebenden äußeren Hülle (2), wobei in der Außenseite Längsrillen(3) angeordnet sind,
**dadurch gekennzeichnet, daß** der Tampon (1) sowohl vor dem Verpressen als auch im ausgedehnten vollgesogenen Zustand eine ovale Querschnittsform besitzt und daß der Tampon im nicht ausgedehnten zusammengepressten Zustand eine zylindrische Form besitzt.

2. Tampon 1 nach Anspruch 1, **dadurch gekennzeichnet, daß** die Anzahl der Längsrillen (3) ungerade ist.

3. Tampon (1) nachAnspruch2,**dadurch gekennzeichnet, daß** er sieben oder neun Längsrillen (3) aufweist.

4. Tampon (1) nach Anspruch 1,**dadurch gekennzeichnet, daß** er im gepressten Zustand am vorderen Ende (Einführende) einen spitzen Kopf aufweist.

## Claims

1. Tampon (1) for female hygiene (menstrual tampon) for introducing into the vagina of a woman with absorbent material made from cellulose fibres and an outer cover (2) surrounding the fibres, in which there are longitudinal grooves (3) on the outside
**characterised by** the fact that before compressing as well as in the expanded, fully absorbed state the tampon (1) has an oval cross section and in the non-expanded, compressed state the tampon has a cylindrical shape.

2. Tampon (1) according to claim 1, **characterised by** the fact that the number of longitudinal grooves (3) is uneven.

3. Tampon (1) according to claim 2, **characterised by** the fact that it has seven or nine longitudinal grooves (3).

4. Tampon (1) according to claim 1, **characterised by** the fact that in the compressed state the front end (leading) is pointed.

## Revendications

1. Tampon (1) pour l'hygiène féminine (tampon menstruel) pour l'introduction dans le vagin d'une femme avec un matériau absorbant en fibres cellulosiques et une gaine (2) extérieure entourant les fibres, des rainures longitudinales (3) étant disposées dans la face extérieure,
**caractérisé par le fait que** le tampon (1) possède une forme ovale en section transversale aussi bien avant la compression qu'également à l'état complètement imbibé dilaté et que le tampon possède à l'état non dilaté compressé une forme cylindrique.

2. Tampon (1) selon la revendication 1,
**caractérisé par le fait que** le nombre des rainures longitudinales (3) est impair.

3. Tampon (1) selon la revendication 2,
**caractérisé par le fait qu'**il présente sept ou neuf rainures longitudinales (3).

4. Tampon (1) selon la revendication 1,
**caractérisé par le fait qu'**il présente, à l'état comprimé, une tête pointue à l'extrémité antérieure (extrémité d'introduction).
